# DEMANDE DE BREVET EUROPEEN

(11) **EP 2 241 269 A1**
(43) Date de publication de la demande: **20.10.2010**
(21) Numéro de dépôt: 10159528.8
(22) Date de dépôt: 09.04.2010
(51) Int. Cl.: A61B 17/12

(54) **Obturateur bronchique à valve**

(30) Priorité: 14.04.2009 FR 0901812; 04.01.2010 FR 1000009
(71) Demandeur: Novatech SA, 13600 La Ciotat (FR)
(72) Inventeur: Miyazawa, M. Hideki, Toyama City Toyama Zip 930-0974 (JP)
(74) Mandataire: Bloch & Bonnétat

(57) **Abrégé**

Selon l'invention, l'obturateur bronchique (1) comprend un corps creux (2), dont la face avant (4) est ouverte et la face arrière (5) comporte au moins une valve (6) fermée dans son état normal, et susceptible d'être volontairement ou spontanément ouverte pour former un orifice de communication. Il comporte en outre des moyens de prise (7) spécialement dédiés à un guide (10), destiné à le mettre en place dans une bronche (16), lesdits moyens de prise (7) étant disposés du côté avant de ladite valve (6) et accessibles à travers ladite valve (6).

## Description

### Obturateur bronchique à valve.

### La présente invention concerne un obturateur bronchique à valve.

On sait que, pour réaliser une embolisation d'une partie d'un poumon atteinte par une fuite d'air dans la plèvre due à un pneumothorax ou à une fistule broncho-pleurale, on peut utiliser des obturateurs bronchiques qui sont introduits, par voie endoscopique, dans les bronches segmentaires ou sous-segmentaires d'un patient. De tels obturateurs sont également utilisés dans les cas d'emphysèmes pulmonaires et pour la réduction du volume pulmonaire.

Par la demande de brevet FR0207268, on connaît un obturateur bronchique qui comporte un corps creux de silicone, de forme approximativement tronconique, dont la face avant (par rapport au sens d'introduction dans les bronches) est plus petite que la face arrière. En outre, la face arrière du corps est ouverte, alors que sa face avant est obturée et porte, sur sa paroi interne, une saillie interne, apte à servir de moyens de prise pour un cathéter destiné à mettre en place ledit obturateur dans une bronche. Ainsi, pour une telle mise en place, le cathéter, ayant saisi ladite saillie interne, pousse l'obturateur à travers les bronches.

Cependant, une fois positionné, l'obturateur bronchique obture intégralement la bronche dans laquelle il a été placé. Aussi, en cas de surpression au sein de la partie aval de la bronche au-delà de la face avant de l'obturateur, il est fréquent que des phénomènes d'hyperinflation apparaissent chez des patients emphysémateux, l'air accumulé dans la partie aval de la bronche ne pouvant s'évacuer en remontant vers la partie amont de celle-ci.

Pour remédier à cet inconvénient, il est connu d'utiliser un obturateur bronchique à valve, qui comprend un corps creux réalisé en matière siliconée élastique, dont la face avant (de nouveau définie par rapport au sens d'introduction dans les bronches) est ouverte et la face arrière comporte une valve unidirectionnelle fermée dans son état normal, et susceptible d'être volontairement ou spontanément ouverte pour former un orifice de communication. Ce dernier, de type fente, est pourvu de deux bords spontanément jointifs, en forme de lèvre plate à la manière d'un bec de canard.

Ainsi, lorsqu'il a été inséré dans une bronche, l'obturateur bronchique peut, en cas de surpression dans la partie aval de la bronche au-delà de sa face avant, supprimer au moins partiellement cette surpression par l'intermédiaire de la valve de sa face arrière, dont les bords spontanément jointifs s'ouvrent élastiquement, pour laisser s'échapper l'air sous pression. Un tel obturateur bronchique a donc pour vocation de se substituer (au moins en première intention) à la réduction du volume pulmonaire par voie chirurgicale.

La mise en place d'un tel obturateur dans une bronche est généralement réalisée par l'intermédiaire d'un guide pourvu à son extrémité distale d'une pince souple, apte à saisir les bords, en forme de lèvre plate, de l'orifice de communication de la face arrière. Une fois les bords saisis, le guide pousse l'obturateur à travers les bronches.

Cependant, étant poussé par sa face arrière, l'obturateur bronchique a tendance, pendant sa mise en place, à se mettre en travers dans lesdites bronches, ce qui rend l'opération difficile, longue et délicate.

De plus, du fait que l'obturateur est en matière souple siliconée, lorsqu'elle saisie les bords de l'orifice de communication de la face arrière, la pince du guide écrase la matière souple desdits bords qui, fréquemment, ne reprend pas sa forme initiale, une fois la pression de la pince relâchée. L'efficacité de la valve est alors dégradée.

La présente invention a pour objet de remédier à ces inconvénients.

A cette fin, selon l'invention, l'obturateur bronchique, comprenant un corps creux, dont la face avant est ouverte et la face arrière comporte au moins une valve fermée dans son état normal, et susceptible d'être volontairement ou spontanément ouverte pour former un orifice de communication, est remarquable en ce que ledit obturateur comporte des moyens de prise spécialement dédiés à un guide, destiné à le mettre en place dans une bronche, et en ce que lesdits moyens de prise sont disposés du côté avant de ladite valve et accessibles à travers ladite valve.

Ainsi, grâce à l'invention, puisque le corps de l'obturateur bronchique est creux et que la face arrière de ce dernier possède une valve, le guide de mise en place peut pénétrer à l'intérieur dudit corps à travers cette valve pour atteindre les moyens de prise, en avant de ladite valve. II en résulte que l'obturateur bronchique n'est plus poussé par l'arrière pendant la mise en place et qu'il n'a plus tendance à se mettre en travers, étant enfilé sur l'extrémité distale du guide.

Afin d'améliorer encore les conditions de mise en place des obturateurs bronchiques, il est avantageux que lesdits moyens de prise soient disposés au voisinage de ladite face avant dudit corps.

Ainsi, pendant l'introduction de l'obturateur bronchique selon l'invention dans une bronche, le maintien de l'obturateur par l'extrémité distale du guide est optimal et le centre de poussée exercée par le guide sur l'obturateur est le plus en avant possible.

Avantageusement, lesdits moyens de prise se présentent sous la forme d'une anse, dont la saisie s'avère aisée. En outre, la forme de l'anse ne perturbe pas, ou très peu, le fonctionnement de l'obturateur bronchique, l'ouverture de la face avant restant dégagée.

De plus, les extrémités longitudinales de ladite anse sont de préférence rapportées (par exemple par collage) sur la paroi interne dudit corps. Bien entendu, en variante, on peut envisager que l'anse et le corps creux de la valve ne forment qu'un seul et même bloc de matière.

Par ailleurs, pour faciliter la mise en place dans une bronche de l'obturateur bronchique, il est avantageux qu'au moins la partie avant dudit corps présente une forme sensiblement tronconique.

De façon préférentielle, ledit orifice de communication, de type fente, comporte deux bords, en forme de lèvre plate, qui se rejoignent spontanément dans l'état normal de ladite valve. Ainsi, les moyens de prise évitent aux bords de l'orifice de communication d'être saisis par le guide, susceptible de les endommager.

Bien entendu, on peut envisager tout autre type d'orifice de communication, dont les bords seraient spontanément jointifs.

En outre, pour améliorer encore l'insertion dans les bronches de l'obturateur bronchique, le contour de ladite face avant comporte avantageusement des échancrures. Ces échancrures permettent de réduire, de façon réversible, la section de ladite face avant pour faciliter l'insertion de l'obturateur dans des bronches de section inférieure à celle du corps creux.

De préférence, ledit corps creux et lesdits moyens de prise sont réalisés en silicone ou en matière analogue. Cependant, en variante, le corps creux et les moyens de prise peuvent être réalisés chacun à partir d'une matière différente.

En outre, le corps creux et les moyens de prise peuvent également être formés par une structure à mailles souple, par exemple métallique, recouverte par un revêtement souple et étanche, par exemple en silicone ou en matière analogue.

Afin d'assurer le maintien correct de l'obturateur selon l'invention dans une bronche, ledit corps creux comporte, sur sa paroi latérale externe, des moyens saillants de maintien en place, par exemple des picots externes aptes à s'appuyer sur la paroi interne de ladite bronche.
Les figures du dessin annexé feront bien comprendre comment l'invention peut être réalisée. Sur ces figures, des références identiques désignent des éléments semblables.
Les figures 1 et 2 sont deux vues en élévation distinctes d'un obturateur bronchique à valve, conforme à la présente invention.
La figure 3 est une coupe axiale, selon la ligne III-III de la figure 1.
La figure 4 est une vue de l'arrière de l'obturateur bronchique de la figure 1, la valve de sa face arrière étant fermée.
La figure 5 est une vue analogue à la figure 4, la valve de l'obturateur bronchique étant ouverte.
Les figures 6A à 6C représentent les différentes étapes de mise en place de l'obturateur bronchique de la figure 1 dans une bronche, au moyen d'un guide à pince.

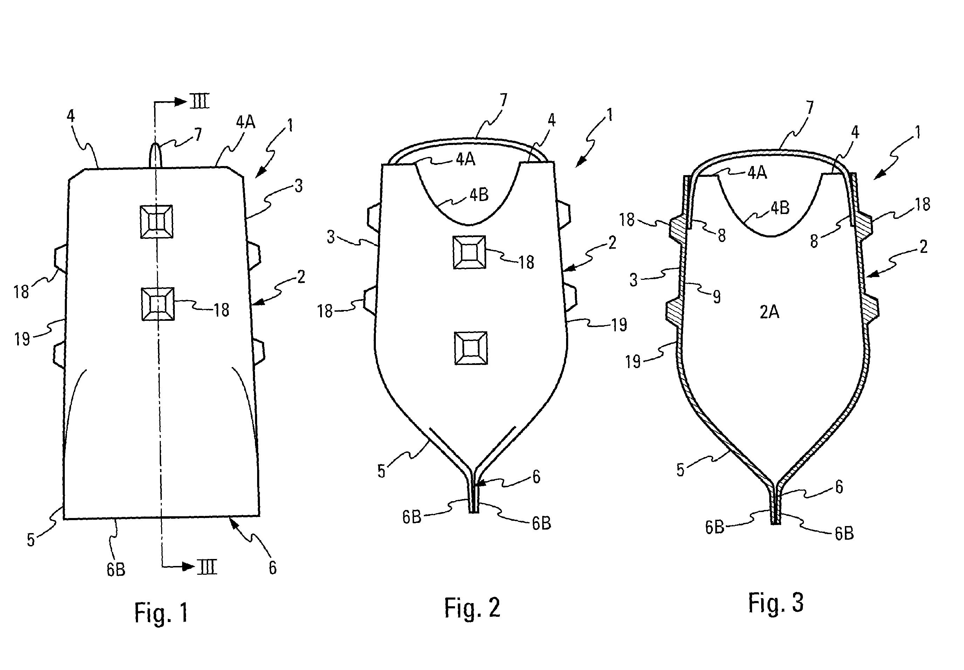

Sur les figures 1 à 3, on a représenté un obturateur bronchique 1 à valve conforme à l'invention. L'obturateur 1 comporte un corps creux 2 de silicone ou de matière analogue, comprenant un évidement intérieur 2A. D'autres matériaux peuvent être utilisés; en particulier, le corps creux 2 peut être formé (au moins partiellement) en titane.

Dans la forme de réalisation présentée, la partie avant 3 du corps creux 2 présente une forme au moins approximativement tronconique. Bien entendu, différentes formes peuvent être prévues pour le corps 2; en particulier, le corps 2 peut être totalement de forme cylindrique, totalement de forme tronconique ou encore comporter une portion de forme cylindrique et une portion de forme tronconique située à l'avant de cette portion cylindrique.

L'obturateur bronchique 1 comporte une face avant 4 (par rapport au sens d'introduction dans les bronches) ouverte et une face arrière 5 qui comporte une valve 6 fermée dans son état normal et susceptible d'être volontairement ou spontanément ouverte pour former un orifice de communication 6A. Les notions d'avant et d'arrière sont fréquemment désignées, dans le domaine des obturateurs bronchiques, par les termes de distal et proximal, respectivement.

L'orifice de communication 6A, de type fente, comporte des bords 6B, en forme de lèvre plate, qui sont maintenus spontanément jointifs dans l'état normal de ladite valve 6.

Selon une forme de réalisation non représentée, la valve 6 comporte, à la base des lèvres 6B, des moyens de renforcement permettant d'éviter une ouverture intempestive des lèvres 6B une fois qu'ils ont été mis en place.

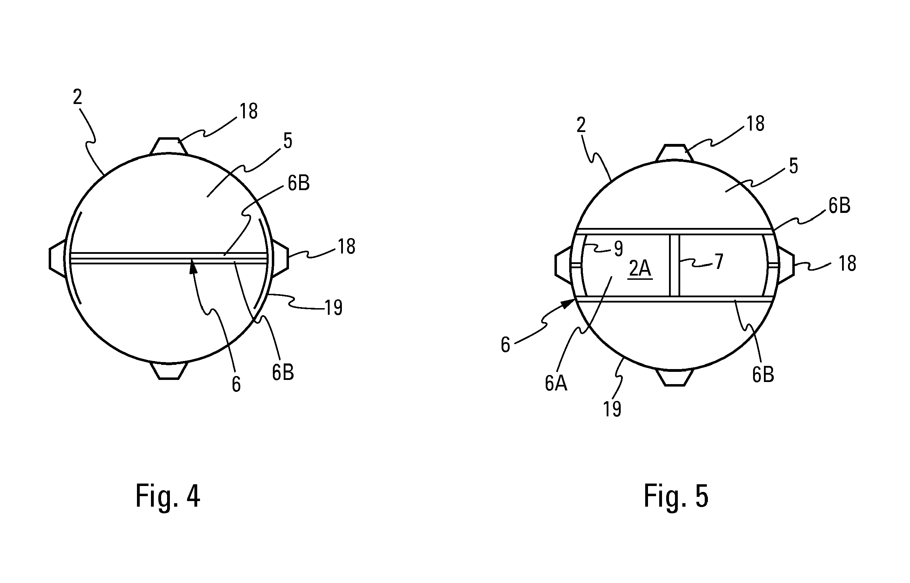

Ainsi, comme le montrent les figures 4 et 5, une fois l'obturateur 1 positionné dans une bronche, lorsque la pression du fluide (généralement de l'air) dans la partie aval de la bronche au-delà de la face avant 4 est au plus égale à la pression dans la partie amont de la bronche au-deçà de la face arrière 5, la valve 6 reste fermée (figure 4), les bords 6B de l'orifice de communication 6A étant maintenus spontanément jointifs grâce à l'élasticité de la matière constitutive de la paroi du corps 2.

En revanche, lorsque la pression du fluide dans la partie aval de la bronche est supérieure à la pression dans la partie amont de la bronche, les bords 6B de l'orifice 6A peuvent s'écarter élastiquement l'un de l'autre en laissant s'échapper du fluide (figure 5). La valve 6 est alors ouverte.

Tel qu'illustré sur les figures 2 et 3, le contour 4A de la face avant 4 comportent deux échancrures 4B en regard, qui permettent de réduire, de façon réversible, la section de la face avant 4 pour faciliter la mise en place de l'obturateur 1 dans une bronche de faible diamètre.

Selon l'invention, tel que représenté sur les figures 1 à 3, l'obturateur bronchique 1 comporte en outre une anse 7 de guidage courbe, par exemple en silicone ou en matière analogue, disposée à l'avant dudit obturateur 1.

Comme le montre la figure 3, les extrémités longitudinales 8 de l'anse de guidage 7 sont rapportées (par exemple par collage) sur la paroi interne 9 du corps creux 2, au voisinage de la face avant 7.

II est bien évident que les extrémités longitudinales de l'anse 7 pourraient également être agencées de toute autre manière désirée (par exemple sur le contour de la face avant de l'obturateur bronchique).

En outre, il est également envisageable de former d'un même bloc de matière l'anse 7 et le corps creux 2 de l'obturateur 1**.**

II est aussi envisageable de former le corps creux 2 et l'anse 7 dans deux matériaux différents, par exemple le corps creux 2 en titane et l'anse 7 en silicone.

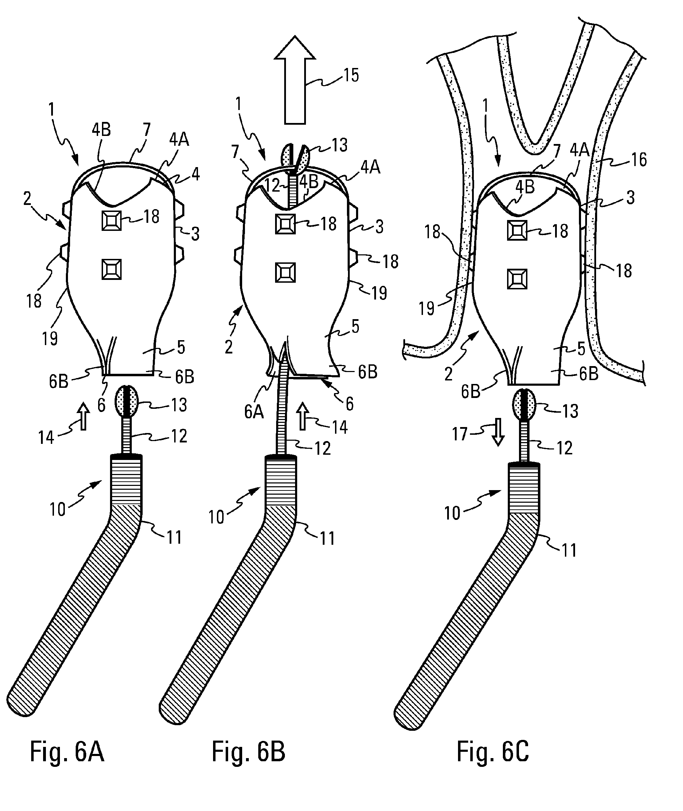

Sur les figures 6A à 6C, on décrit la mise en place de l'obturateur bronchique 1, conforme à l'invention, par l'intermédiaire d'un guide 10. Ce dernier comporte un cathéter 11 (par exemple un fibroscope) dans lequel peut se déplacer une tige de guidage 12 pourvue, à son extrémité distale, d'une pince souple 13, l'ouverture et la fermeture de cette dernière étant commandées par un opérateur.

Tout d'abord, tel qu'illustré par les figures 6A et 6B, on déploie la tige de guidage 12 hors du cathéter 11 (le déplacement vers l'avant de la tige 12 est repéré par la flèche 14), pour qu'elle puisse s'introduire dans l'évidement 2A du corps creux 2, après avoir traversée la valve 6 de la face arrière 5 de l'obturateur 1, et atteindre l'anse de guidage 7. Ensuite, la pince souple 13 peut s'ouvrir pour saisir l'anse de guidage 7.

Lorsque l'anse de guidage 7 a été saisie par la pince 13, le déploiement de la tige de guidage 12 peut se poursuivre, de façon à pousser vers l'avant l'obturateur 1, comme indiqué par la flèche 15. II en résulte que le centre de poussée de la tige de guidage 12 du guide 10 est situé à l'avant de l'obturateur bronchique 1, de sorte que le suivi de la trajectoire d'avance par l'obturateur 1 est optimal.

Comme le montre la figure 6C, lorsque l'obturateur bronchique 1 a été correctement positionné dans une bronche 16, la tige de guidage 12 est rentrée dans le cathéter 11 (le déplacement vers l'arrière de la tige 12 est symbolisé par la flèche 17), de manière à l'extraire de l'obturateur 1.

En outre, des picots 18 saillants (par exemple tronconiques) portés par la paroi latérale externe 19 de l'obturateur bronchique 1, permettent de maintenir ledit obturateur 1 en place à l'intérieur de la bronche 16 (figure 6C). Tout autre moyen de maintien adapté peut être prévu, en particulier toute structure ou élément en saillie hors de la surface externe de la paroi latérale 19 du corps 2 de l'obturateur 1, par exemple un ou plusieurs anneau(x) circonférentiel(s).

## Revendications

1. Obturateur bronchique comprenant un corps creux (2), dont la face avant (4) est ouverte et la face arrière (5) comporte au moins une valve (6) fermée dans son état normal, et susceptible d'être volontairement ou spontanément ouverte pour former un orifice de communication (6A),
**caractérisé en ce que** ledit obturateur (1) comporte des moyens de prise (7) spécialement dédiés à un guide (10), destiné à le mettre en place dans une bronche (16), et **en ce que** lesdits moyens de prise (7) sont disposés du côté avant de ladite valve (6) et accessibles à travers ladite valve (6).

2. Obturateur bronchique selon la revendication 1,
**caractérisé en ce que** lesdits moyens de prise (10) sont disposés au voisinage de ladite face avant (4) dudit corps (2).

3. Obturateur bronchique selon l'une des revendications 1 ou 2, **caractérisé en ce que** lesdits moyens de prise (7) se présentent sous la forme d'une anse.

4. Obturateur bronchique selon la revendication 3,
**caractérisé en ce que** les extrémités longitudinales (8) de ladite anse (7) sont rapportées sur la paroi interne (9) dudit corps (2).

5. Obturateur bronchique selon l'une des revendications 1 à 4, **caractérisé en ce qu'**au moins la partie avant (3) dudit corps (2) présente une forme sensiblement tronconique.

6. Obturateur bronchique selon l'une des revendications 1 à 5, **caractérisé en ce que** ledit orifice de communication (6A), de type fente, comporte deux bords (6B), en forme de lèvre plate, qui se rejoignent spontanément dans l'état normal de ladite valve (6).

7. Obturateur bronchique selon l'une des revendications 1 à 6, **caractérisé en ce que** le contour (4A) de ladite face avant (4) comporte des échancrures (4B).

8. Obturateur bronchique selon l'une des revendications 1 à 7, **caractérisé en ce que** ledit corps creux (2) et lesdits moyens de prise (7) sont réalisés en silicone ou en matière analogue.

9. Obturateur bronchique selon l'une des revendications 1 à 8, **caractérisé en ce que** ledit corps creux (2) comporte, sur sa paroi latérale externe (19), des moyens saillants (18) de maintien en place, par exemple des picots externes (18) de section circulaire ou en forme de losange.
